Europäisches Patentamt

European Patent Office

Office européen des brevets

(11) Veröffentlichungsnummer: **0 093 897**
**A1**

## (12) EUROPÄISCHE PATENTANMELDUNG

(21) Anmeldenummer: **83103686.8**

(22) Anmeldetag: **15.04.83**

(51) Int. Cl.³: **A 61 B 6/04,** A 61 B 5/10, A 61 B 6/00

(30) Priorität: **30.04.82 DE 3216273**

(43) Veröffentlichungstag der Anmeldung: **16.11.83** Patentblatt **83/46**

(84) Benannte Vertragsstaaten: **DE FR NL**

(71) Anmelder: **SIEMENS AKTIENGESELLSCHAFT, Berlin und München Wittelsbacherplatz 2, D-8000 München 2 (DE)**

(72) Erfinder: **Naser, Georg, Karlstrasse 10, D-8502 Zirndorf (DE)**
Erfinder: **Reichenberger, Helmut, Dr., Begonienstrasse 28, D-8501 Eckental (DE)**
Erfinder: **Schneider, Siegfried, Dr., Eskilstunastrasse 27, D-8520 Erlangen (DE)**

(54) **Medizinische Anordnung für die Diagnose und/oder Therapie.**

(57) Eine medizinische Einrichtung für die Diagnose und/oder Therapie, bestehend aus einer Lagerstatt (2, 102) für einen Patienten und einem zugehörigen Applikator (17, 117) mit Betriebsgerät (16, 116). Die Lagerstatt (2, 102) weist wenigstens einen Sensor (5, 105, 109) zur Registrierung und Erkennung von Körpervorgängen, wie z.B. Atmungs- bzw. Herzaktionen, auf. Die Sensorsignale werden in einer Signalverarbeitungseinheit (14, 21–85, 121–156) ausgewertet und zur Steuerung der Diagnose- und/oder Therapiemaßnahmen dem Betriebsgerät (16, 116) für den Applikator (17, 117) zugeführt.

SIEMENS AKTIENGESELLSCHAFT
Berlin und München

Unser Zeichen
VPA 82 P 3733 E

0093897

## Medizinische Anordnung für die Diagnose und/oder Therapie

Die Erfindung bezieht sich auf eine medizinische Anordnung für die Diagnose und/oder Therapie, bestehend aus einer Lagerstatt für einen Patienten und einem Applikator mit Betriebsgerät und zugehörigen Diagnose- und/oder Therapieeinrichtungen.

Bei Untersuchungen und Behandlungen von Patienten, die längere Zeit benötigen, ist eine sichere und kontrollierbare Lagerung des Patienten von Bedeutung. Dies gilt beispielsweise für eine herkömmliche Röntgendurchstrahlung, aber auch insbesondere für neuere bildgebende Verfahren wie die verschiedenen tomographischen Methoden sowie Ultraschall- oder Nukleardiagnostik. Während bei der klassischen Röntgendurchstrahlung die Belichtungszeiten meist so kurz sind, daß durch Patientenmotorik oder Organbewegungen kaum Artefakte auftreten werden, sind letztere bei den tomographischen Methoden nicht vernachlässigbar. Bei der Computer-Tomographie ist man derzeit unter anderem aus diesem Grund bemüht, insbesondere die Meßzeiten zu verringern. Während bei der Röntgen-Computer-Tomographie inzwischen Meßzeiten von weniger als 5 sec erreichbar sind, ist dies aber beispielsweise bei der Kernspin-Tomographie aus systembedingten Gründen nicht möglich. Hier betragen die Untersuchungszeiten minimal 30 sec.

Wht 5 Rl / 8.4.1982

Weiterhin besteht häufig der Wunsch ein Bild zu bestimmten Zeitpunkten im Ablauf eines periodischen, physiologischen Vorganges, wie z.B. Atmung oder Herzaktion, zu erhalten. Auch Aufforderungen an den Patienten, sich nicht zu bewegen oder den Atem anzuhalten lassen sich aus verschiedenen Gründen, wie beispielsweise bei Kindern oder bewußtlosen Patienten häufig nicht realisieren. Schon alle normalen physiologischen Vorgänge können demzufolge zu Artefakten führen. Das gleiche gilt insbesondere für die durch solche physiologische Vorgänge verursachten Bewegungen von inneren Organen. Auch läßt sich bei längeren Untersuchungen die Motorik des Patienten nicht im wünschenswerten Maße ausschließen.

Abgesehen von den reinen Diagnoseverfahren ist eine definierte und kontrollierbare Patientenlagerung auch bei den verschiedensten therapeutischen Verfahren wichtig. Dies gilt wiederum beispielsweise für eine herkömmliche Strahlentherapie, aber auch insbesondere für solche neueren Therapieverfahren wie beispielsweise die lokale Hyperthermie durch externe Bestrahlung oder die Nierensteinzertrümmerung mit externen Stoßwellen. Bei diesen therapeutischen Verfahren kommt es immer darauf an, die Therapiewirkung auf eng umschriebene Organbereiche zu begrenzen. Innere Organe werden aber - wie bereits erwähnt - beispielsweise durch die Atmung periodisch um Strecken bis zu einigen Zentimetern verschoben. So ist z.B. für die exakte Lokalisierung eines durch Stoßwellen zu zerstörenden Steines in der Niere eine laufende Kontrolle durch eine Stereoröntgenanlage notwendig.

Es ist bereits bekannt (Radiology 140 (1981) S. 413 bis 420), Computer-Tomographie-Aufnahmen des mensch-

lichen Herzens durch EKG-Signale zu triggern. Des weiteren ist insbesondere aus der US-PS 42 89 142 ein Monitor für physiologische Ereignisse, beispielsweise für Atmung- oder auch Herzaktionssignale, vorbekannt, bei dem ebenfalls Triggersignale für die Auslösung eines Röntgengerätes od.dgl. geliefert werden können. Speziell dort geht es allerdings im wesentlichen um die Aufbereitung der mittels Körperelektroden erhaltenen elektrischen Signalkurven. Des weiteren ist aus der US-PS 39 93 995 ein solcher Monitor für die Atmungstätigkeit eines Patienten, insbesondere eines Säuglings, bekannt, bei dem Mikrowellen auf den Thorax-Abdominalbereich des Patienten eingestrahlt werden und aus der Phasenverschiebung der reflektierten Signale Trigger zur Betätigung eines Röntgengerätes abgeleitet werden. Bei der US-PS 39 93 995 wird als besonders vorteilhaft herausgestellt, daß die Signale berührungslos erhalten werden. Allerdings ist eine derartige Signalaufnahme vergleichsweise unsicher. Strahlungsquelle und Strahlungsempfänger müssen in spezifischer Weise auf den Patienten ausgerichtet werden, wobei insbesondere bei Änderungen der Körperlage des Patienten das Signal verlorengehen und eine Nachjustierung notwendig werden kann.

Aufgabe der Erfindung ist es daher, bei einer Anordnung der eingangs genannten Art Maßnahmen anzugeben, mit denen während des Betriebes des Applikators möglicherweise auftretende Artefakte kontrollierbar und ausschließbar sind, ohne daß spezifische Sensoren am Körper des Patienten angebracht oder auf diesen ausgerichtet werden müssen. Dabei sollen insbesondere die von Artefakten befreiten Signale entsprechend den physiologischen Körpervorgängen zur Steuerung des Betriebsgerätes herangezogen werden können.

Die Aufgabe wird erfindungsgemäß dadurch gelöst, daß die Lagerstatt wenigstens einen Sensor zur Erfassung mechanischer Körpervorgänge aufweist, dessen Signale in einer Signalverarbeitungseinheit ausgewertet und für eine mit den mechanischen Körpervorgängen gekoppelte Steuerung der Diagnose- und/oder Therapiemaßnahmen dem Betriebsgerät für den Applikator zugeführt werden.

Gemäß der Erfindung sind also zur Erzeugung der Triggersignale weder Körperelektroden anzubringen noch solche speziellen Strahlungseinrichtungen auf den Patientenkörper auszurichten und gegebenenfalls nachzuführen. Vielmehr ist unmittelbar als Bestandteil der Lagerstatt bereits ein Sensor zur Erfassung mechanischer Körpervorgänge, der keinen zusätzlichen Applikationsaufwand benötigt, vorgesehen.

Besonders vorteilhaft ist bei der Erfindung, daß sich nunmehr wesentliche mechanische Körpervorgänge während der Patientenlagerung kontinuierlich erfassen lassen. Das so gewonnene Primärsignal läßt sich in die unterschiedlichen Frequenzbereiche aufspalten und jeweils der Motorik, der Atmung oder den Herzaktionen des Patienten zuordnen. Durch einfache logische Verknüpfungen lassen sich Steuergrößen für die Aktivierung des Betriebsgerätes des diagnostischen oder therapeutischen Applikators gewinnen.

Je nach Anwendungszweck ist es nun beispielsweise denkbar, standardisierte oder patientenindividuelle Triggerkriterien durch den Anwender vorzugeben, welche als Grundlage für die Signalverarbeitung dienen. Es ist also somit die Möglichkeit gegeben, sowohl die durch die mechanischen Körpervorgänge hervorgerufenen

0093897

Arteiakte zu eliminieren als auch den Ablauf dieser
Körpervorgänge selbst zur Steuerung der diagnostischen
oder therapeutischen Maßnahmen heranzuziehen.

Weitere Einzelheiten und Vorteile der Erfindung ergeben sich aus der nachfolgenden Figurenbeschreibung
von Ausführungsbeispielen anhand der Zeichnung.

Es zeigen:

Fig. 1 eine erste erfindungsgemäße Anordnung, bei
der die Lagerstatt in der Horizontalebene
fixiert ist,

Fig. 2 die zugehörige Auswerteschaltung,

Fig. 3 mechanographische Signalkurven zur Erläuterung der Fig. 2,

Fig. 4 eine zweite erfindungsgemäße Anordnung mit
schwenkbarer Lagerstatt und

Fig. 5 einen Ausschnitt aus der zugehörigen Auswerteschaltung.

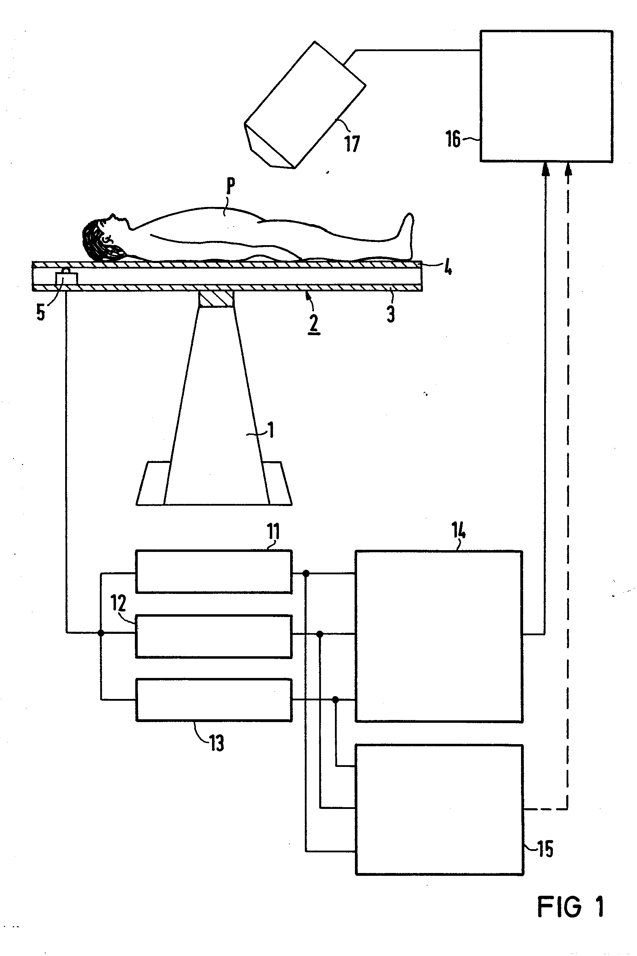

In der Figur 1 ist schematisch eine Patientenlagerungseinrichtung mit den zugehörigen medizinischen
Diagnose- und/oder Therapieeinrichtungen dargestellt:
Mit 1 ist ein Stativfuß bezeichnet, auf dem in geeigneter Höhe eine Lagerstatt 2 fixiert ist. Die
Lagerstatt 2 besteht im wesentlichen aus zwei parallelen
Platten 3 und 4 zwischen denen ein Kraftsignalgeber 5
angeordnet ist. Damit wirkt die gesamte Lagerstatt aus
den beiden Platten 3 und 4 einschließlich des Kraftsignalgebers 5 als Sensor für die Aufnahme von einwirkenden Kräften bzw. Kraftänderungen. Der Kraftsignalgeber 5 ist in etwa in der Höhe des Kopfbereiches der Lagerstatt 2 angebracht. Auf der Liegefläche 3 der Lagerstatt 2 ist ein Patient mit P angedeutet.

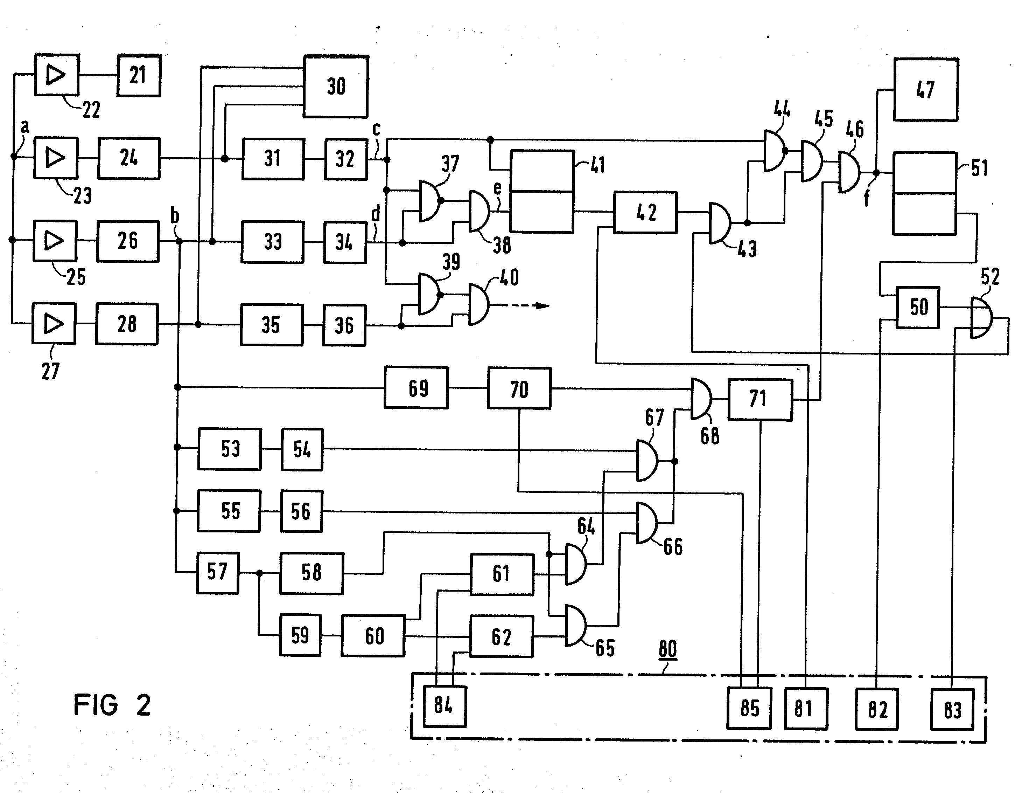

Das Signal des Kraftsignalgebers 5 gelangt über drei elektronische Filtereinheiten 11 bis 13 zu einer Signalverarbeitungsschaltung 14. Diese Signalverarbeitungsschaltung wird weiter unten anhand der Figur 2 im einzelnen erläutert.

In den Filtereinheiten 11 bis 13 wird das Signal des Kraftsignalgebers 5 frequenzmäßig getrennt und der Motorik, der Atmung, sowie Heraktionen zugeordnet. Die Einzelsignale sind auf dem Display eines Oszilloskops 15 bzw. eines Schreibers od.dgl. darstellbar.

Von der Signalverarbeitungsschaltung 14 bzw. dem Oszilloskop 15 sind Triggersignale auf ein Betriebsgerät 16 rückkoppelbar. Vom Betriebsgerät 16 wird ein Applikator 17 angesteuert. Ein solcher Applikator kann im einzelnen für eine Diagnose oder Therapie ausgelegt sein. Er kann beispielsweise eine herkömmliche Röntgenröhre sein.

In der Figur 2 ist mit 21 eine Einheit bezeichnet, die ein vom Kraftsignalgeber bewirktes elektrisches Signal liefert. Der Einheit 21 ist ein Verstärker 22 nachgeschaltet, dessen Ausgangssignal in drei Parallelzweige aufgeteilt wird. In einem ersten Zweig folgt einem einstellbaren Verstärker 23 ein elektrisches Filter 24 zur Ausfilterung der Motoriksignale. In einem zweiten Zweig folgt einem einstellbaren Verstärker 25 ein Filter 26 zur Ausfilterung von Atmungssignalen. In einem dritten Zweig folgt einem Verstärker 27 ein Filter 28 zur Ausfilterung von Herzaktionssignalen. Die Ausgangssignale können auf einem Oszilloskop 30 dargestellt werden; davon unabhängig gelangen sie zur eigentlichen Auswerteschaltung gemäß Pos. 14 der Figur 1.

In der Auswerteschaltung wird speziell die Verarbeitung des Atemsignals und dessen Verknüpfung mit dem Motoriksignal zur Erzeugung der Triggerfunktion ausgeführt. Auf die Darstellung einer entsprechenden Schaltung für die Herzaktionen wird nicht näher eingegangen, da auf der Basis der EKG-Abnahme bereits ebenfalls praktikable Triggerverfahren bekannt sind.

Den einzelnen Zweigen der Signalverarbeitungsschaltung ist jeweils ein Schwellenglied mit nachgeschaltetem Triggerglied zugeordnet. Im einzelnen bezeichnen 31 das Schwellenglied für ein Motoriksignal und 32 den ersten Motoriktrigger, 33 ein Schwellenglied für das Atmungssignal und 34 den ersten Atmungstrigger sowie 35 ein Schwellenglied für das Herzaktionssignal und 36 den ersten Herzaktionstrigger. Von den Triggersignalen werden jeweils paarweise logische Schaltglieder angesteuert und zwar UND-NICHT-Glieder 37 bzw. 39 und UND-Glieder 38 bzw. 40. In der vorliegenden Schaltung bewirkt dies, daß am Ausgang dann ein Triggersignal ansteht, wenn eine Atmungsaktion bzw. eine Herzaktion, aber keine Motorikaktion auftritt. Die zugehörigen Triggersignale sind mit $TR_2$ und $TC_2$ bezeichnet.

Vom Triggersignal TR$_2$ wird ein rücksetzbarer Zähler 41 angesteuert. Der Zähler 41 zählt die Triggersignale auf, wobei jeweils ein Motoriksignal das Zurücksetzen des Zählers 41 auf Null bewirkt. Dem Zähler 41 ist ein Komparator 42 nachgeschaltet, der den Zählerstand mit einem vorgegebenen Sollwert vergleicht. Hierzu weist ein Anzeigepaneel 80 der Auswerteschaltung betätigbare Einstellglieder auf, von denen die Einheit für die Zählervorgabe mit 81 bezeichnet ist.

Das Ausgangssignal des Komparators 42 wird über mehrere logische Schaltglieder gegeben, durch die sichergestellt wird, daß auch bei während Verzögerungszeiten noch auftretende Motoriksignale ein Zurücksetzen bewirkt wird. Im einzelnen bedeuten 43 ein UND-Glied, 44 ein UND-NICHT-Glied sowie 45 und 46 UND-Glieder. Über die UND-Glieder 43 und 46 können weitere Bedingungen in die logische Schaltung    eingekoppelt werden.

Wenn am Ausgang des UND-Gliedes 46 ein Signal ansteht, wird das Gerät für die Diagnose und/oder Therapie, das die Kennziffer 47 hat, eingeschaltet. Gleichzeitig wird vom Signal des UND-Gliedes 46 eine Uhr 51 auf Null gesetzt und gestartet. Dies ist dann von Bedeutung, wenn beispielsweise bestimmte Mindestpausenzeiten zwischen aufeinanderfolgenden Triggern eingehalten werden sollen. Dafür ist ein Komparator 50 vorhanden, der die abgelaufene Zeit jeweils mit einem Sollwert des entsprechenden Sollwertgebers 82 im Anzeigepaneel 80 vergleicht. Vom Komparator 50 wird ein ODER-Glied 52 zusammen mit dem Signal einer Einschalteinheit 83 angesteuert, von wo das Ausgangssignal auf das UND-Glied 43 zurückgekoppelt wird.

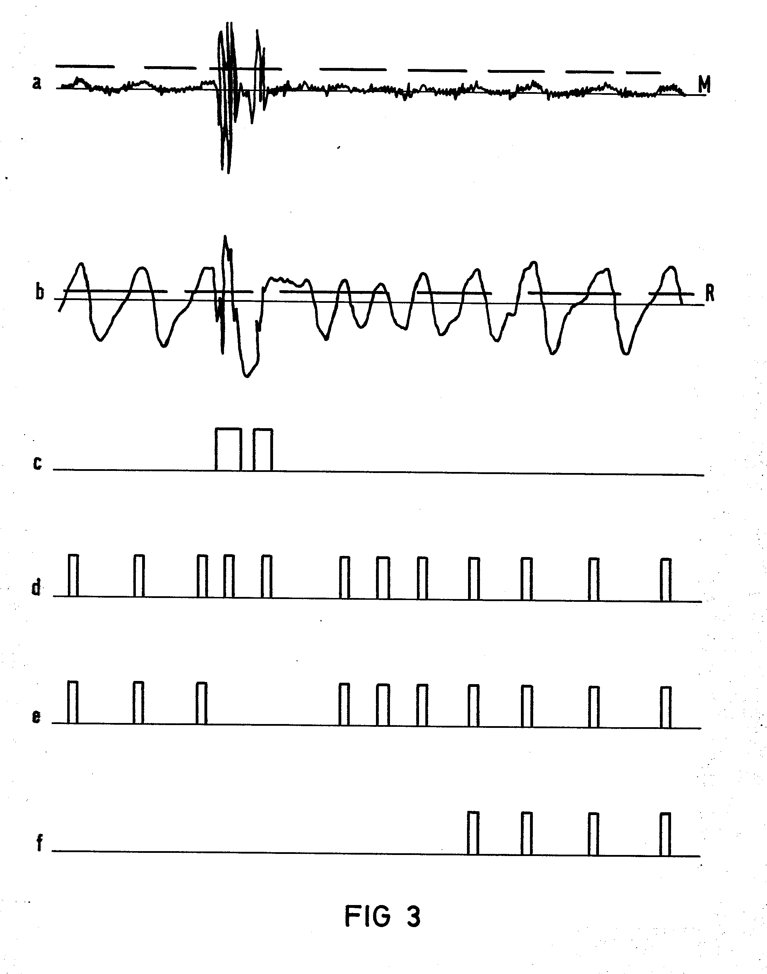

Speziell für die Auswertung des Atmungssignals lassen sich aus der Atmungskurve verschiedene Bedingungen ableiten, welche auch den Diagrammen der Figur 3 im einzelnen entnehmbar sind. Dafür wird das Atmungssignal in einem ersten Zweig auf ein Schwellenglied 53 zur Bestimmung einer unteren Schwelle gegeben, von der ein Trigger 54 ein Triggersignal ableitet. In einem parallelen Zweig wird entsprechend mit einem weiteren Schwellenglied 55 ein oberes Schwellensignal gebildet und von einem Trigger 56 ein Triggersignal aus dem oberen Schwellensignal abgeleitet.

Mittels zweier Differenzierglieder 57 und 59 und zugehöriger Detektoren kann im einzelnen die Atemphase bestimmt werden. Dies ist dann von Bedeutung, wenn beispielsweise eine Diagnose und/oder Therapie mit der Inspiration bzw. Exspiration getriggert erfolgen soll. Dem Differentiator 57 ist ein Null-Detektor 58 zugeordnet, dem Differentiator 59 ein Vorzeichen-Detektor 60. In Verbindung mit nachfolgenden Komparatoren 61 und 62 lassen sich bei Anwahl an einer Einheit 84 des Anzeigepaneels 80 entsprechende Atemphasen vorwählen. Die Signale der Kompratoren 61 und 62 werden zusammen mit dem Signal des Null-Detektors 58 über UND-Glieder 64 und 65 sowie 66 und 67 derart verknüpft, daß ein Signal für die obere bzw. untere Schwelle bzw. für das Ein- und Ausatmen erhalten wird. Wenn man davon ausgeht, daß vom Wechsel der Atemphase das Aktivierungssignal mit vorgegebener Verzögerung erfolgen soll, so wird weiter über einen Null-Detektor 69 und ein Verzögerungsglied 70 ein Signal geliefert, das durch ein UND-Glied 68 mit den Signalen der Schwellentrigger verknüpft wird. Das entsprechende Verzögerungssignal kann wiederum an einer Sollwerteinheit 85 am Anzeigepaneel 80 vorgewählt werden. Das Signal des UND-Gliedes 68 wird über ein weiteres

Verzögerungsglied auf das UND-Glied 71 gegeben. Mit der Schaltungsgruppe 53 bis 71 ist also eine Vorwahl des Triggerzeitpunktes für den Applikator 17 in bezug auf den Ablauf der Respiration möglich.

In der Figur 3 ist die Anwendung der Auswerteschaltung gemäß Fig. 2 auf die mechanographischen Signale eines Kindes gezeigt,  die mittels eines im Bereich der Lagerstatt angeordneten Kraftsignalgebers aufgenommen werden. In den mechanographischen Signalkurven sind die entsprechenden Schwellenwerte eingezeichnet: Im einzelnen  sind in den ersten beiden Zeilen Signalkurven, die mit der erfindungsgemäßen Anordnung aufgenommen wurden, dargestellt. Zeile a zeigt eine signifikante Signalkurve (nach Einheit 22) mit von einem Patienten bewirkten mechanischen Lebensfunktionen, die deutliche Motorikaktionen zeigt, aus der speziell in Zeile b die Atmungsaktionen (nach Einheit 26) herausgefiltert sind. In beiden Zeilen sind Schwellenwerte M bzw. R eingezeichnet.

In den Zeilen c bis f sind nun die durch die Auswerteschaltung nach Figur 2 ableitbaren Trigger für entsprechende Steuerungszwecke dargestellt: Zeile c (nach Einheit   32) liefert entsprechend der in Zeile a eingestellten Schwelle zwei Triggersignale; ganz entsprechend werden in Zeile d (nach Einheit  34) Triggersignale für die Atmung geliefert. Wenn man nun davon ausgeht, daß der Applikator für die Diagnose und/oderTherapie von Atmungsaktionen gesteuert werden soll, lassen sich über die logische Verknüpfung solche Atmungstrigger ausschließen, die von einer Motorikaktion überlagert sind. Dies ist in Zeile e (nach Einheit  38 ) dargestellt. Beispielsweise kann nun mittels Sollwertgeber ein  Zählersollwert

von vier Atmungssignalen eingestellt werden, mit denen
die diagnostischen und/oder therapeutischen Maßnahmen
gestartet werden. Dies ergibt sich in beispielhafter
Form aus der Zeile f, welche die an das Betriebsgerät
für den Applikator gelangenden Triggersignale (nach
Einheit 46) wiedergibt.

Durch Differentiation der Atemkurve lassen sich ganz
entsprechend solche Signalkurven ableiten, aus denen
die Inspirations- bzw. Exspirationsphasen entnehmbar
sind. Daraus können wieder mittels spezieller logischer
Verknüpfung der Figur 2 spezifische Triggerbedingungen
abgeleitet werden.

Entsprechendes gilt auch für Herzaktionssignalkurven.
Beispielsweise lassen sich hier durch Verknüpfung von
Motorik, Atmungs- und Herzaktionskurven solche Triggerbedingungen ableiten, bei denen die Diagnose- und/oder
Therapiemaßnahmen durch Ausschaltung von Motorik und
Respiration allein durch die Herzaktion gesteuert
werden.

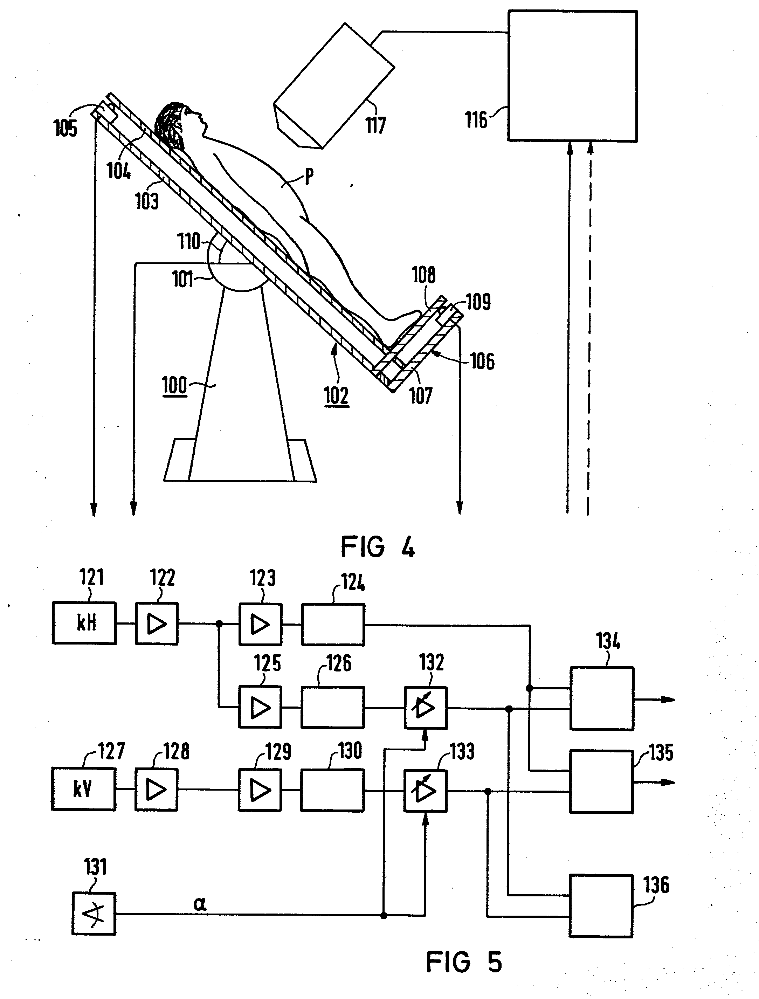

In der Figur 4 ist auf einem Stativ 100 mit Schwenkeinrichtung 101 eine Lagerstatt 102 verkippbar angeordnet. Die Lagerstatt 102 besteht wiederum aus zwei
parallel angeordneten Platten 103 und 104, wobei dazu senkrecht ein Fußteil 106 aus ebenfalls zwei
parallelen Platten 107 und 108 angeordnet ist. Zwischen die parallelen Platten 103, 104 sowie 107, 108
ist ganz entsprechend der Figur 1 ein erster Kraftsignalgeber 105 sowie ein zweiter Kraftsignalgeber 109
angeordnet, so daß von den Stützflächen jeweils wieder
Sensoren für Kraftänderungen gebildet werden. Im
Schwenklager 101 befindet sich weiterhin ein Winkelgeber 110. Entsprechend Figur 1 ist ein Betriebsgerät
mit 116 und ein Applikator für die Diagnose oder
Therapie mit 117 bezeichnet.

Bei einer solchen Einrichtung lassen sich mit dem ersten (horizontalen) Kraftsignalgeber 105 insbesondere die Motorik und Atmungssignale, mit dem zweiten (vertikalen) Kraftsignalgeber 109 dagegen speziell die Herzaktionssignale erfassen, da letztere im wesentlichen Kraftwirkungen vorzugsweise in Längsrichtung des Patientenkörpers bewirken.

In der zugehörigen Auswerteschaltung nach Figur 5 ist dem horizontalen Kraftsignalgeber 121 ein Verstärker nachgeschaltet, aus dessen Signal Motorik- und Respirationssignale abgeleitet werden. 123 kennzeichnet einen Verstärker für das Motoriksignal und 124 ein entsprechendes Filter während 125 einen Verstärker für das Respirationssignal mit entsprechendem Filter 126 kennzeichnet. Der Einheit für den vertikalen Kraftsignalgeber 127 ist ebenfalls ein Verstärker 128 nachgeschaltet, aus welchem Signal wieder in bekannter Weise mittels eines Verstärkers 129 und entsprechenden Filters 130 das Herzaktionssignal abgeleitet wird. Vom Winkelgeber 131 gesteuert sind nun beide Signale über einstellbare Verstärker 132 und 133 selektiv veränderbar. Das heißt also, es kann bei der Signalauswertung die momentane Stellung der Lagerstatt berücksichtigt werden.

Weiterhin bedeuten 134 eine Triggereinheit für das Respirationssignal sowie 135 eine Triggereinheit für das Herzaktionssignal. Des weiteren ist ein Oszilloskop 136 ansteuerbar. Von den Ausgangssignalen der Trigger 134 und 135 kann entweder wiederum eine logische Schaltung mit Verknüpfungsgliedern entsprechend der Figur 2 oder auch unmittelbar das medizinische Gerät angesteuert werden.

Die Funktion der vorstehend anhand der Figur 2 und 5 beschriebenen Auswerte- bzw. Triggerschaltungen kann zumindest teilweise software-mäßig realisiert werden. Dazu eignet sich z.B. ein Rechnersystem, bei dem ein Programmspeicher patientenindividuell programmierbar ist, so daß die Steuerung des Applikators für die Diagnose oder Therapie problemspezifisch realisiert werden kann.

5 Figuren
13 Patentansprüche

0093897

Patentansprüche

1. Medizinische Anordnung für die Diagnose und/oder Therapie, bestehend aus einer Lagerstatt für einen Patienten und einem Applikator mit Betriebsgerät und zugehörigen Diagnose- und/oder Therapieeinrichtungen, dadurch gekennzeichnet , daß die Lagerstatt (2, 102) wenigstens einen Sensor (5, 105, 109) zur Erfassung mechanischer Körpervorgänge aufweist, dessen Signale in einer Signalverarbeitungseinheit (14, 21-85, 121-136) ausgewertet und für eine mit den mechanischen Körpervorgängen gekoppelte Steuerung der Diagnose- und/oder Therapiemaßnahmen dem Betriebsgerät (16, 116) für den Applikator (17, 117) zugeführt werden.

2. Anordnung nach Anspruch 1, dadurch gekennzeichnet , daß die Liege- und/oder Stützflächen der Lagerstatt (2, 102) doppelwandig ausgeführt sind, wobei wenigstens ein Kraftsignalgeber (5, 105, 109) zur Aufnahme der vom Patienten verursachten Kraftänderungen zwischen beiden die Liege- und/oder Stützflächen (2, 102) bildenden Wandflächen (3, 4; 103, 104; 107, 108) angeordnet ist.

3. Anordnung nach Anspruch 2, dadurch gekennzeichnet , daß ein erster Kraftsignalgeber (5, 105) im Kopfbereich der Liegefläche (4, 104) der Lagerstatt (2, 102) angeordnet ist.

4. Anordnung nach Anspruch 2, dadurch gekennzeichnet , daß ein zweiter Kraftsignalgeber (109) in einer senkrecht zur Liegefläche (104) stehenden Stützfläche (106) der Lagerstatt (102) angeordnet ist.

5. Anordnung nach ~~den vorliegenden Ansprüchen~~ einem der vorherstehenden Ansprüche, wobei speziell zwei Kraftsignalgeber vorhanden sind, von denen der eine im Bereich der Liegefläche und der andere im Bereich der Stützfläche angeordnet ist, d a d u r c h   g e k e n n z e i c h n e t , daß die Lagerstatt (102) auf dem Stativ (101, 102) kippbar ist und eine Meßeinrichtung (110) zur Erfassung des Kippwinkels ($\alpha$) aufweist.

6. Anordnung nach Anspruch 5, d a d u r c h   g e k e n n z e i c h n e t , daß in der Signalverarbeitungseinheit (121-136) die Signale der Kraftsignalgeber (105, 109) abhängig von der Winkelstellung der Lagerstatt (102) verarbeitet werden.

7. Anordnung nach einem der vorhergehenden Ansprüche, d a d u r c h   g e k e n n z e i c h n e t , daß die Signalverarbeitungseinheit (21-85, 121-136) Frequenzfilter (11-13; 24, 26, 28) und einstellbare Verstärker (23, 25, 27) zur Trennung und Aufbereitung des ursprünglichen Signals in der Motorik, Atmung und/oder Herzaktion zuord**bare** Signale aufweist.

8. Anordnung nach Anspruch 7, d a d u r c h   g e k e n n z e i c h n e t , daß aus wenigstens einem der aufbereiteten Signale Steuergrößen für das Betriebsgerät (16, 47, 116) des Applikators (17, 117) abgeleitet werden.

9. Anordnung nach Anspruch 8, d a d u r c h   g e k e n n z e i c h n e t , daß Schwellenglieder (31, 33, 35; 53, 55) zum Setzen vorwählbarer Schwellenwerte für die Signale vorhanden sind.

10. Anordnung nach Anspruch 8, d a d u r c h   g e - k e n n z e i c h n e t ,   daß mittels Sollwertgeber (80) Sollwerte für die Signale vorwählbar sind.

11. Anordnung nach Anspruch 8, d a d u r c h   g e - k e n n z e i c h n e t ,   daß Verknüpfungsglieder (37-40, 43-46, 65-68) die Signale verschiedener Kanäle verknüpfen und daraus Triggersignale für das Betriebsgerät (16, 47, 116) abgeleitet werden.

12. Anordnung nach Anspruch 11, d a d u r c h   g e - k e n n z e i c h n e t ,   daß speziell das Atemsignal in bezug auf Kriterien zur Beurteilung des Atemzustandes, beispielsweise für die Endwerte von Inspiration oder Exspiration bzw. Atempause vorgebbarer Dauer, ausge- wertet und bei Erfüllung der Kriterien entsprechende Triggersignale ausgelöst werden.

13. Anordnung nach Anspruch 7, d a d u r c h   g e - k e n n z e i c h n e t ,   daß die Signalverarbeitung zumindest teilweise von einer Rechnereinheit durchge- führt wird.

FIG 1

FIG 2

FIG 3

FIG 4

FIG 5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | DE-A-2 158 457 (A.G. BROWN, BOVERI & CIE.) <br> * Seite 3, Zeile 6 - Seite 4, Zeile 23; Seite 7, Zeile 21 - Seite 8, Zeile 11; Seite 9, Zeilen 5-23; Seite 10, Zeilen 12-16; Seite 11, Zeilen 14-22; Seite 12, Zeilen 13-16; Abbildungen 1-4 * | 1,8,9, 11 | A 61 B 6/04 <br> A 61 B 5/10 <br> A 61 B 6/00 |
| A | FR-A-1 432 775 (M.J.A. CARA) <br> * Seite 1, linke Spalte, Zeilen 24-30; Seite 2, linke Spalte, Zeile 49 - rechte Spalte, Zeile 31; Abbildungen 1,3 * | 2-4,7 | |
| A | US-A-3 890 958 (L.P. FISTER et al.) <br> * Zusammenfassung; Spalte 5, Zeilen 26-42; Spalte 12, Zeile 66 - Spalte 13, Zeile 26; Spalte 14, Zeilen 26-56; Abbildungen 4-9, 12-15 * | 2,9,11 ,13 | RECHERCHIERTE SACHGEBIETE (Int. Cl. 3) <br><br> A 61 B 6/04 <br> A 61 B 5/10 <br> A 61 B 6/00 |
| A | US-A-3 822 875 (W. SCHMEDEMANN) <br> * Zusammenfassung; Spalte 2, Zeilen 23-28; Spalte 2, Zeile 65 - Spalte 3, Zeile 5; Spalte 3, Zeilen 32-46; Abbildungen 1-5 * <br><br> --- -/- | 5,6 | A 61 B 5/08 |

Der vorliegende Recherchenbericht wurde für alle Patentansprüche erstellt.

| Recherchenort | Abschlußdatum der Recherche | Prüfer |
|---|---|---|
| DEN HAAG | 08-08-1983 | RIEB D.K. |

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | FR-A-2 041 477 (THOMSON MEDICAL-TELCO) * Seite 1, Zeile 21 - Seite 2, Zeile 22; Seite 3, Zeilen 17-20; Seite 4, Zeilen 13-21; Abbildungen 1,2 * | 7-9,11 ,12 | |
| | --- | | |
| A | FR-A-2 273 505 (INSERM) * Seite 2, Zeile 28 - Seite 3, Zeile 11; Seite 4, Zeilen 10-16; Abbildungen 1-4 * | 7-12 | |
| | ----- | | |
| | | | RECHERCHIERTE SACHGEBIETE (Int. Cl. ³) |

Der vorliegende Recherchenbericht wurde für alle Patentansprüche erstellt.

| Recherchenort DEN HAAG | Abschlußdatum der Recherche 08-08-1983 | Prüfer RIEB D.K. |
|---|---|---|